# EUROPEAN PATENT SPECIFICATION

(11) **EP 0 617 964 B1**
(45) Date of publication and mention of the grant of the patent: **21.08.2002**
(21) Application number: 93919688.7
(22) Date of filing: 20.09.1993
(51) Int. Cl.: A61K 33/06, A61K 33/10, A61K 31/7004, A61K 31/7016, A61K 31/19, A61K 47/02, A61K 9/08

(54) **INJECTION FOR CURING INJURED ABNORMAL TISSUE, PROCESS FOR PRODUCING THE SAME, AND USE THEREOF**
INJEKTION ZUR HEILUNG VON VERLETZTEM ABNORMEM GEWEBE, VERFAHREN ZU IHRER HERSTELLUNG UND IHRE VERWENDUNG
INJECTION POUR SOIGNER DES TISSUS ALTERES LESES, PROCEDE POUR SA PRODUCTION, ET SON UTILISATION

(30) Priority: 18.09.1992 JP 27513292
(43) Date of publication of application: 05.10.1994
(73) Proprietor: LEQUIO PHARMA CO., LTD., Naha-shi, Okinawa (JP); Mitsubishi Pharma Corporation, Osaka-shi, Osaka 541-0046 (JP)
(72) Inventor: OKAZAKI, Hideo, Higashiyamato-shi, Tokyo 189 (JP)
(74) Representative: Gille Hrabal Struck Neidlein Prop Roos
(86) International application number: JP9301347
(87) International publication number: WO94006443

(56) References cited:
- EP-A- 0 342 524
- JP-A- 4 225 920
- US-A- 4 395 398
- US-A- 4 626 433

## Description

### TECHNICAL FIELD

The present invention relates to an injectable preparation for curing affected abnormal tissues, a method for the preparation thereof, and usage thereof and, more particularly, to a stable injectable preparation for curing affected abnormal tissues caused, for example, by brain aneurysm, hemorrhoids, hepatic tumor vessels, and the like, a method for the preparation thereof, and usage thereof.

### BACKGROUND ART

Heretofore, oil-based phenol preparations have been administered by injection as a procedure for curing hemorrhoids. This procedure, however, is short in sustaining the effects upon the curing of the hemorrhoids and the hemorrhoids are caused to occur again in six months to two years on the average. Hence, strong demands have been made to develop pharmaceutical preparations that can be injected under observations by the anoscope for curing particularly severe hemorrhoids.

On the other hand, currently, there is no appropriate pharmaceutical preparations for combating against the brain aneurysm and it is treated by surgical operations such as craniotomy. Recent years, abnormality of cerebral blood vessels can be detected in increasing cases as image diagnose devices such as CT (computer scanning diagnose device), MRI (magnet resonance image computer scanning diagnose device) and the like have developed greatly and employed extensively. Hence, pharmaceutical preparations in liquid form which can prevent the rupture of the brain aneurysm have been desired.

In addition, fluid agents for obstructing blood vessels, such as granular gelatin sponge, fibrinogen and the like, are employed for curing affected abnormal tissues caused by, for example, hepatic tumor. The effects achieved by the treatment of such agents for obstructing blood vessels, however, cannot be said sufficient and satisfactory. In many cases, the affected abnormal tissues caused by the hepatic tumor are treated by the hepatic lobules exsection by laparotomy. Hence, strong demands have been made to develop liquid agents which are effective for the treatment of the affected abnormal tissues caused by, for example, the hepatic tumor vessels and the like and which can be employed in place of the conventional agents for obstructing the blood vessels.

As described hereinabove, the conventional pharmacotherapy for curing the affected abnormal tissues caused by, for example, brain aneurysm, hemorrhoids, hepatic tumor vessels and the like cannot achieve satisfactory treatment effects and cannot in many cases suppress the symptoms thereof from occurring again.

In order to satisfy such demands, a composition comprising gallnut, alum and glycerin is proposed as an agent for curing the hemorrhoids (Journal of Traditional Chinese Medicine (1981): 1(2), 87-92). Such a composition for curing the hemorrhoids cannot be employed as an injectable solution. Even if the composition would be intended to be employed as injectable preparations, the problems may arise particularly with respect to stability, resulting in coloring or, in some cases, depositing during storage for a long period of time. As a matter of fact, the use of tannic acid in combination with potassium aluminium sulfate is inhibited as disclosed in "Pharmacopedia Japonica". Hence, the greatest care is required to be paid to storing such a composition so that it is very inconvenient for storage. Further, if the composition would be employed as an injectable solution, investigation with respect to coloring or depositing should be made with the greatest care before administration. This is very inconvenient for administering the composition as an injectable solution. In addition, the problem may further arise that the composition may be decided as inappropriate as an injectable solution as a result of investigation.

Furthermore, Japanese Patent Unexamined Publication Kokai No. 4-225,920 discloses, as an agent for hardening the affected tissues of the digestive system such as, for example, esophagophlangioma, hemorrhoids, the site of the rectal prolapse, the site of the prolapse of the rectal mucosa and the affected uplift tissues of the large intestine and the rectum, a composition comprising tannic acid and potassium aluminium sulfate and having an extract of a medicinal plant, containing a phenol, a flavone or a flavonoid, a catechin or a polycarboxylic acid, as a stabilizing agent. As this composition contains the extract of the medicinal plant as a stabilizing agent, however, it suffers from the disadvantages in terms of formulating preparations that it is extremely difficult to provide injectable prepara-tions containing a stabilizing agent with a constant formulation and that there is still the possibility that the stabilizing agent may be contaminated with minute amounts of unidentified substances even if it would be extracted and purified through elaborately plural steps. It has further been found that even a composition comprising tannic acid and potassium aluminium sulfate may cause coloring or, in rare cases, depositing, even if it contains the extract of the plant as a stabilizing agent, when stored for a long period of time. This also may cause the problem when it is applied as an injectable solution.

### DISCLOSURE OF INVENTION

Therefore, the present invention has the object to provide an injectable preparation in a solution form for curing the affected abnormal tissues, particularly which can solve the problems that conventional compositions for curing the affected abnormal tissues or the hardening agents for hardening the affected tissues of the digestive system, as described hereinabove, are instable during storage for a long period of time and which can sustain its stability as pharmaceutical preparations during storage for a long period of time as well as ensure effectiveness in addition to stability.

The present invention has another object to provide an injectable preparation in a solution form for curing the affected abnormal tissues, which also can effectively be adapted to the affected abnormal tissues caused by, for example, the brain aneurysm, hemorrhoids, hepatic tumor vessels and the like, which could not be cured by conventional pharmacotherapy.

The present invention has a further object to provide a method for the production of an injectable preparation in the solution form for curing the affected abnormal tissues caused by the brain aneurysm, hemorrhoids, hepatic tumor vessels and the like, which causes no problems with properties as pharmaceutical preparations even during storage for a long period of time and which can ensure stability as well as effectiveness.

The present invention is further related to a use of an injectable preparation in the solution form for curing the affected abnormal tissues, which can cause no problems with preparations during storage for a long period of time and which can ensure stability as well as effectiveness.

Other objects, features and advantages of the present invention will become apparent in the course of the description of the preferred embodiments.

### BEST MODE FOR CARRYING OUT THE INVENTION

The injectable preparation in a solution form for curing the affected abnormal tissues in accordance with the present invention comprises at least a water-soluble aluminium compound, tannic acid and sodium hydrogen sulfite as an antioxidant. In addition, the injectable preparation in the solution form may contain a polyvalent alcohol or a saccharide as an agent for raising osmosis of the curing injectable preparation for curing the affected abnormal tissues caused by, for example, brain aneurysm, hemorrhoids and hepatic tumor.

As the water-soluble aluminium compound to be employed for the composition for curing the affected abnormal tissues in accordance with the present invention, there may be mentioned, for example, aluminium chloride, aluminium sulfate, aluminium carbonate, aluminium acetate, aluminium nitrate, aluminium lactate, aluminium tartrate, aluminium salicylate, sodium aluminium sulfate, potassium aluminium sulfate, cesium aluminium sulfate, ammonium aluminium sulfate and the like. The water-soluble aluminium compound may be applied singly or in combination of two or more.

The water-soluble aluminium compound is formulated in concentrations ranging usually from 0.01 to 0.5 mole, preferably from 0.03 to 0.3 mole. If the water-soluble aluminium compound would be contained in a concentration beyond the above range, the resulting injectable solution may cause coloring or depositing during storage and it becomes inappropriate as injectable preparations and it cannot accomplish the desired effects sought to be achieved by the present invention.

The tannic acid to be employed for the curing injectable solution according to the present invention may be contained at a rate ranging usually from 0.05% to 10.0%, preferably from 1.0% to 5.0%, with respect to the total weight of the ingredients of the injectable solution, excluding distilled water. The rate of the tannic acid with respect to the water-soluble aluminium compound may range usually from 0.5% to 25.0%, preferably from 1.0% to 20.0%. If the tannic acid would be contained in a concentration beyond the above range, the resulting injectable solution may cause coloring or depositing during storage for a long period of time and it becomes inappropriate as injectable preparations and it cannot accomplish the desired effects sought to be achieved by the present invention.

In addition, sodium hydrogen sulfite to be employed as the antioxidant for the curing injectable solution according to the present invention may be contained at a rate ranging usually from 0.05% to 0.5%, preferably from 0.1% to 0.3%, with respect to the total weight of the ingredients of the injectable solution, excluding distilled water. If the tannic acid would be contained in a concentration beyond the above range, the resulting injectable solution cannot accomplish the desired effects sought to be achieved by the present invention.

As the polyvalent alcohol or the saccharide to be employed as an agent for raising the osmosis of the curing injectable solution for curing the affected abnormal tissues in accordance with the present invention, there may be mentioned, for example, mannitol, fructose, xylitol, glucose, galactose, mannose, lactose, glycerin and the like. The polyvalent alcohol and/or saccharide may be employed singly or in combination of two or more. The polyvalent alcohol and/or saccharide may be contained so as to raise the osmosis of the resulting injectable solution by approximately three times to fifteen times, preferably from approximately four times to eight times, that of physiological saline, and the amount of the polyvalent alcohol and/or saccharide is not limited as long as the osmosis of the injectable solution is raised to that level.

In addition, the curing injectable solution according to the present invention may contain a chelating agent such as sodium citrate at a rate ranging usually from 0.1% to 5.0%, preferably from 1.0% to 5.0%.

In accordance with the present invention, it is of significance from the point of view of storage of the injectable solution, too, that a pH of the composition is maintained in a range usually from pH1.5 to pH3.5, preferably from pH2.0 to pH3.0. When the ingredients of the composition are prepared by the method according to the present invention, the resulting injectable solution can usually have the liquid properties as defined hereinabove so that no adjustment of the liquid properties of the injectable solution is required in usual cases. If needed, however, the resulting compositions can be adjusted by using a pharmacologically non-toxic acid or alkali, which has hitherto been employed for the same purposes, including a mineral acid, such as hydrochloric acid, sulfuric acid or the like or sodium hydrogen carbonate, sodium hydroxide or the like, in order to provide the liquid properties in the ranges as described hereinabove. When the resulting compositions have the liquid properties within the ranges as described hereinabove, they are extremely stable as preparations without causing coloring or any depositing during storage even for a long period of time.

Further, the injectable preparations according to the present invention may contain another ingredient which has hitherto been employed as an ingredient for injectable preparations, such as an agent for increasing viscosity of a solution, e.g. dextran or the like, or an antiseptic agent, e.g. phenol, benzyl alcohol, benzarconium chloride, p-aminobenzoic acid ester or the like. These ingredients may optionally been employed within the range that does not substantially affect the effects of the injectable preparations adversely.

The compositions containing the above ingredients can be prepared for injectable preparations by means of conventional methods for the preparation of injectable preparations. For example, the injectable solution can be prepared by dissolving each of the ingredients one after another in distilled water or by dissolving or suspending some of all the ingredients in distilled water and then dissolving the rest of the ingredients in the resulting solution or suspension. Then, the resulting injectable composition is usually sterilized by filtration and filled in colorless hard glass ampoules or glass bottles. They are then sterilized by high-pressure steam, as needed, and stored in cold place. The injectable compositions according to the present invention may be employed by dissolving the ingredients in distilled water in site on account of their ingredients. The liquid properties of the injectable compositions are generally adjusted so as to have the pH of the injectable preparation within the above-defined range at an appropriate time prior to filling the compositions into ampoules, vials or bottles. Dissolved oxygen present in the injectable preparations can be removed by conventional methods and the injectable preparations are then replenished with nitrogen gas.

The injectable preparations according to the present invention will be described in more detail by way of formulations.

### Preparation Formulation Example No. 1:

| | |
|---|---|
| Potassium aluminium sulfate | 400 mg |
| Tannic acid | 15 mg |
| Sodium citrate | 150 mg |
| Dextran 40 | 70 mg |
| Sodium hydrogen sulfite | 15 mg |
| Glycerin | 1,000 mg |
| Injectable water | To make 10 ml |

The given amounts of potassium aluminium sulfate, tannic acid, sodium citrate, dextran, sodium hydrogen sulfite and glycerin were placed in a test tube and injectable water was added to the mixture of the ingredients so as to make the volume of the mixture 10 ml and the mixture was dissolved by stirring to give a solution. The pH of the resulting solution was found to be pH2.7. The solution was then filled in glass bottles, followed by degasification of dissolved oxygen and then by replenishment of air with nitrogen gas. Thereafter, the injectable compositions were sterilized by high-pressure steam and stored in cold place.

### Preparation Formulation Example No. 2:

| | |
|---|---|
| Aluminium chloride | 400 mg |
| Tannic acid | 15 mg |
| Sodium citrate | 150 mg |
| Dextran 40 | 70 mg |
| Sodium hydrogen sulfite | 15 mg |
| Mannitol | 1,500 mg |
| Injectable water | To make 10 ml |

The given amounts of aluminium chloride, tannic acid, sodium citrate, dextran, sodium hydrogen sulfite and mannitol were placed in a test tube and injectable water was added to the mixture of the ingredients so as to make the volume of the mixture 10 ml and the mixture was dissolved by stirring to give a solution. The pH of the resulting solution was adjusted with hydrochloric acid to pH3.0. The solution was then filled in glass bottles, followed by degasification of dissolved oxygen and then by replenishment of air with nitrogen gas. Thereafter, the injectable compositions were sterilized by high-pressure steam and stored in cold place.

### Preparation Formulation Example No. 3:

| | |
|---|---|
| Aluminium sulfate | 400 mg |
| Tannic acid | 15 mg |
| Sodium citrate | 150 mg |
| Dextran 40 | 70 mg |
| Sodium hydrogen sulfits | 15 mg |
| Fructose | 2,000 mg |
| Injectable water | To make 10 ml |

The given amounts of aluminium sulfate, tannic acid, sodium citrate, dextran, sodium hydrogen sulfite and fructose were placed in a test tube and injectable water was added to the mixture of the ingredients so as to make the volume of the mixture 10 ml and the mixture was dissolved by stirring to give a solution. The pH of the resulting solution was adjusted with sulfuric acid to pH2.5. The solution was then filled in glass bottles, followed by degasification of dissolved oxygen and then by replenishment of air with nitrogen gas. Thereafter, the injectable compositions were sterilized by high-pressure steam and stored in cold place.

### Preparation Formulation Example No. 4:

| | |
|---|---|
| Aluminium acetate | 200 mg |
| Tannic acid | 15 mg |
| Sodium citrate | 150 mg |
| Dextran 40 | 70 mg |
| Sodium hydrogen sulfite | 15 mg |
| Xylitol | 2,000 mg |
| Injectable water | To make 10 ml |

This preparation formulation was prepared in substantially the same manner as preparation formulation example No. 1 and the pH of the resulting composition was adjusted with sulfuric acid to pH2.5.

### Preparation Formulation Example No. 5:

| | |
|---|---|
| Aluminium carbonate | 200 mg |
| Tannic acid | 15 mg |
| Sodium citrate | 150 mg |
| Dextran 40 | 70 mg |
| Sodium hydrogen sulfite | 15 mg |
| Glucose | 3,000 mg |
| Injectable water | To make 10 ml |

This preparation formulation was prepared substantially in the same manner as the preparation formulation example No. 1 and the pH of the resulting composition was adjusted with sulfuric acid to pH2.7.

### Preparation Formulation Example No. 6:

| | |
|---|---|
| Potassium aluminium sulfate | 400 mg |
| Tannic acid | 75 mg |
| Sodium citrate | 150 mg |
| Dextran 40 | 70 mg |
| Sodium hydrogen sulfite | 15 mg |
| Glycerin | 1,000 mg |
| Injectable water | To make 10 ml |

The given amounts of potassium aluminium sulfate, tannic acid, sodium citrate, dextran, sodium hydrogen sulfite and glycerin were placed in a test tube and injectable water was added to the mixture of the ingredients so as to make the volume of the mixture 10 ml and the mixture was dissolved by stirring to give a solution. The pH of the resulting solution was adjusted with sulfuric acid to pH2.7. The solution was then filled in glass bottles, followed by degasification of dissolved oxygen and then by replenishment of air with nitrogen gas. Thereafter, the injectable compositions were sterilized by high-pressure steam and stored in cold place.

### Comparative Preparation Formulation Example No. 1:

The preparation formulation Example No. 1 was adjusted with sodium hydroxide to pH4.7.

### Comparative Preparation Formulation Example No. 2:

The preparation formulation was prepared in the same manner as the preparation formulation example No. 1 by excluding sodium citrate and sodium hydrogen sulfite from the ingredients of the preparation formulation example No. 1, and the resulting preparation formulation was adjusted with sulfuric acid to pH2.7.

### Comparative Preparation Formulation Example No. 3:

| | |
|---|---|
| Potassium aluminium sulfate | 400 mg |
| Tannic acid | 15 mg |
| Sodium citrate | 150 mg |
| Dextran | 100 mg |
| Chlorobutanol | 50 mg |
| Glycerin | 1 mg |
| Injectable water | To make 10 ml |

The above ingredients were prepared in the same manner as above and the resulting preparation formulation was adjusted with sodium hydroxide to pH4.7.

### Comparative Preparation Formulation Example No. 4:

The preparation formulation was prepared in the same manner as the preparation formulation example No. 3 by excluding sodium citrate and sodium hydrogen sulfite from the ingredients of the preparation formulation example No. 3, and the resulting preparation formulation was adjusted with sulfuric acid to pH2.7.

### Comparative Preparation Formulation Example No. 5:

The preparation formulation as disclosed in Japanese Patent Unexamined Publication Kokai No. 4-225,920 as Preparation Example No. 1 was prepared in the same manner, and the resulting composition was adjusted with sodium hydroxide to pH4-5. Colorless hard glass vials were filled with 10 ml of the resulting aqueous solution. After replenishment of gases present in the vials with nitrogen gases, the vials were sterilized by high-pressure steam in conventional manner.

### Comparative Preparation Formulation Example No. 6:

The preparation formulation as disclosed in Japanese Patent Unexamined Publication Kokai No. 4-225,920 as Preparation Example No. 2 was prepared in the same manner, and the resulting composition was adjusted with sodium hydroxide to pH4-5. Colorless hard glass vials were filled with 10 ml of the resulting aqueous solution. After replenishment of gases present in the vials with nitrogen gases, the vials were sterilized by high-pressure steam in conventional manner.

### Comparative Preparation Formulation Example No. 7:

The preparation formulation as disclosed in Japanese Patent Unexamined Publication Kokai No. 4-225, 920 as Preparation Example No. 5 was prepared in the same manner, and the resulting composition was adjusted with sulfuric acid to pH2.7. Colorless hard glass vials were filled with 50 ml of the resulting aqueous solution. After replenishment of gases present in the vials with nitrogen gases, the vials were sterilized by high-pressure steam in conventional manner.

The injectable solutions as prepared in the manner as described hereinabove are then tested for their properties by preparations stability tests, acute toxicity tests, tests for the repair of brain aneurysm in monkeys, tests for curing hemorrhoids in monkeys, and tests for curing hepatic tumor in dogs.

### Preparations Stability Tests:

After the injectable preparation formulations prepared in the above-mentioned Examples were adjusted to given pH ranges, five colorless hard glass vials were aseptically filled with 10 ml of each of the injectable preparations and replenished with nitrogen gas under vacuum conditions. The vials were then exposed to white light with 1,000 lux at 40° C and visual observations were made as to coloring and the extent of depositing. Before the tests for stability of preparations, each of the injectable preparations was found colorless or transparent, yellowish and slightly viscous. The test results are shown in Table 1 below.

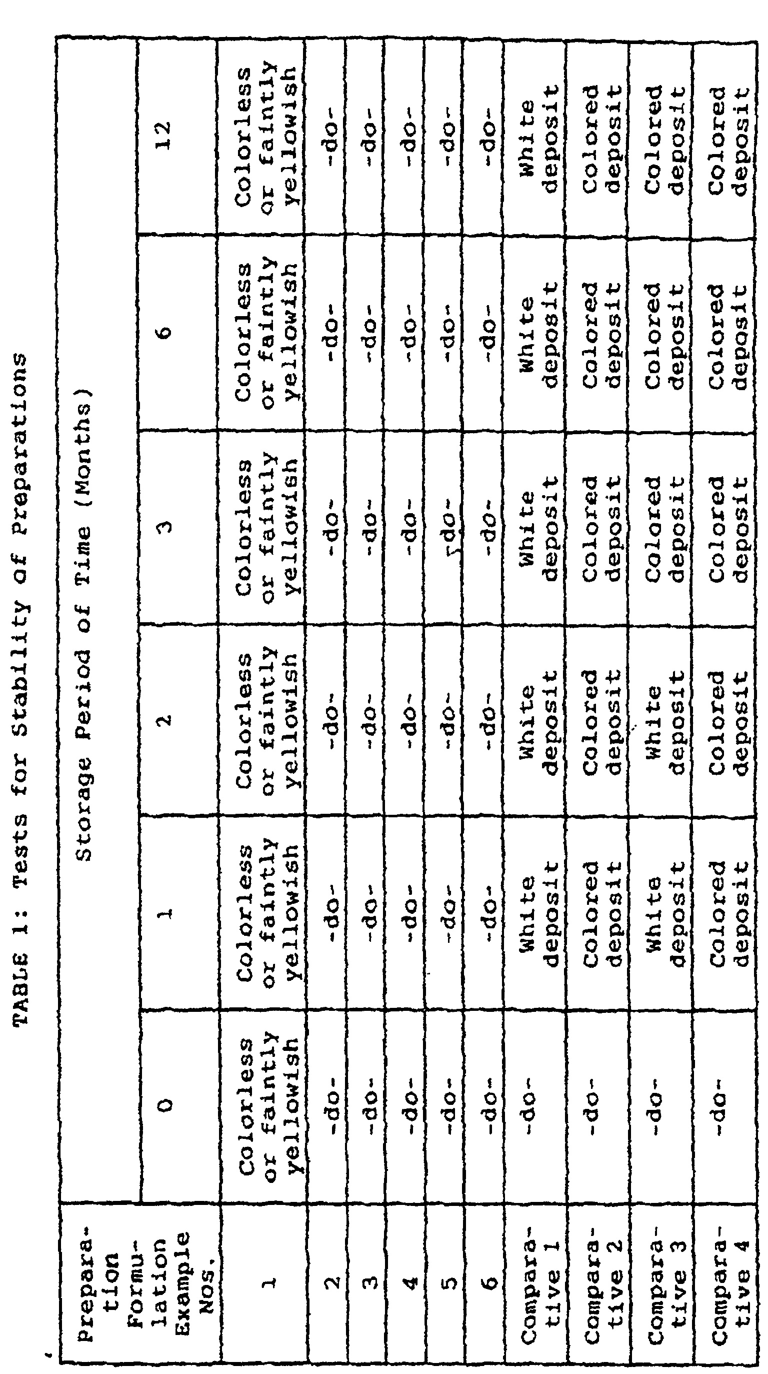

Further, the comparative preparation formulation example Nos. 5 and 7 prepared by the procedures as described in Japanese Patent Unexamined Publication Kokai No. 4-225,920 were decided as inappropriate for clinical administration because their formulations were unstable with respect to their compositions because of the extract of medicinal plants. Hence, they were excluded from the tests for stability of the preparations.

In addition, the comparative preparation formulation Examples Nos. 5 to 7 prepared by the procedures as disclosed in Japanese Patent Unexamined Publication Kokai No. 4-225,920 were decided as inappropriate as injectable preparations because they contain edetoate.

### Acute Toxicity Tests:

The injectable preparation formulation example No. 1 was administered intravenousually (iv) or intraperitoneally (ip) to four groups of rats, each group consisting of ten male Wister-type rats weighing 200 ± 20 grams. The results of death were observed in fourteen days after administration and the lethal dose (LD50), i.e. the rate of a half of experimental animals died, was computed in accordance with the Richfield-Wilcockson method. It was found that the lethal dose (LD50) was 8.3 ± 0.5 ml/kg when it was administered intravenousually (iv) and that the lethal dose (LD50) was 30 ml/kg or more when it was administered intraperitoneally (ip).

### Tests for Repair of Brain Aneurysm by Monkeys:

The injectable solutions according to the present invention were tested in order to confirm whether it is effective for the repair of the brain aneurysm in monkeys.

The tests for repairing the brain aneurysm were carried out by using healthy nine monkeys with 10 years or more of age and the nine monkeys were divided into three groups of each three. Before the tests, each monkey was subjected to encephalo-arteriography and it was confirmed that each had aneurysm having a size as large as its fifth finger in section.

To the first group of monkeys as a control, there was slowly and gradually injected a solution containing equivalent amounts of physiological saline and an iodo contrast medium at the rate of 0.1 ml/kg over the period of 10 minutes through a catheter introduced into the affected part through its arteriae carotis interna.

To the second group of monkeys, there was slowly and gradually injected a solution containing equivalent amounts of the preparation formulation example No. 1 and the iodo contrast medium at the rate of 0.1 ml/kg through a catheter in the same manner as above.

To the third group of monkeys, there was slowly and gradually injected a solution containing the preparation formulation example No. 1 and the two-fold amount of the iodo contrast medium at the rate of 0.1 ml/kg through a catheter in the same manner as above.

As a result of these tests, it was found that the ability of contrasting was slightly reduced when the contrast medium was diluted. No clinical problems, however, could be seen when the encephalo-arteriography was carried out using a digital substraction angiography device (DSA). Although it is of importance that no symptom of the defluxion of the nerves is caused to occur for the repair of the brain aneurysm, in addition to the normalization of the blood vessels at their swollen parts, it was found that the concentration of the injectable solution served as a particularly significant factor in order to achieve the effects of causing no such symptom to occur. The test resuts are shown in Table 2 below.

**TABLE 2:**

| Test for Repairing Brain Aneurysm in Monkeys | | | | | |
|---|---|---|---|---|---|
| Group | No. | Abnormal Angiograph | Curing Effects | Symptom of Defluxion of Nerves | Comprehensive Effects |
| 1 | 1 | No change | None | None | Not effective |
| | 2 | -do- | -do- | -do- | -do- |
| | 3 | -do- | -do- | -do- | -do- |
| 2 | 1 | Remarkably improved | Remarkably improved | Recognized | -do- |
| | 2 | -do- | -do- | -do- | -do- |
| | 3 | -do- | -do- | -do- | -do- |
| 3 | 1 | -do- | -do- | None | Remarkable |
| | 2 | -do- | -do- | -do- | -do- |
| | 3 | -do- | -do- | -do- | -do- |

In order to confirm the effectiveness of the curing injectable solution according to the present invention for curing the hemorrhoids, the tests for curing the hemorrhoids have been carried out in a manner as will be described hereibelow.

### Tests for Curing Hemorrhoids in Monkeys:

A hemorrhoid model in monkeys was formed in a manner as will be described below. in 10 ml of an injectable water was dissolved 0.1 mg of commercially available dry snake toxin, and a histolysis agent was prepared by adding 10 mg of hyaluronidase, 100 mg of chondroitin sulfate and a very small amount of epinephrine to the solution. Six monkeys with one year of age were divided into three groups of each two, and 3 ml of the histolysis agent was administered once per week to each monkey in the submucosa and the mucoderm of its rectum and the anal tract. The monkeys were fed with high protein feed with less fibrous materials, thereby reducing the intestinal vermiculation. Each of the monkeys was raised in a cage with a ring put on around its neck and their legs standing straight. As they grew in this state, they become always in a state of constipation and become accustomed to defecation with anger and the stool became small in amount and hard. The anal portion of each monkey was turned into a congestive state upon repetition of the defecation with anger, and the proctoptosis was caused upon defecation. This completed the hemorrhoid model in monkey.

Each monkey in the first group as a control was injected in the blood vessel knobs of its hemorrhoids with 10 ml of physiological saline. In the second group, eah monkey was injected in the blood vessel knobs of its hemorrhoids with 10 ml of an injectable solution prepared by diluting the preparation formulation example No. 1 with an equivalent amount of physiological saline. In the third group, eah monkey was injected in the blood vessel knobs of its hemorrhoids with 10 ml of an injectable solution prepared by diluting the preparation formulation example No. 6 with an equivalent amount of physiological saline. The test results are shown in Table 3 below.

**TABLE 3:**

| Tests for Curing Hemorrhoids in Monkeys | | | |
|---|---|---|---|
| Group | Monkey Nos. | Blood Vessel Knobs of Hemorrhoids | Curing Effect |
| 1 | 1 | Not Changed | Not Effective |
| | 2 | -do- | -do- |
| 2 | 1 | Disappeared | Remarkably Effective |
| | 2 | -do- | -do- |
| 3 | 1 | -do- | -do- |
| | 2 | -do- | -do- |

In order to confirm the effectiveness of the curing injectable solution according to the present invention for curing the hepatic tumor, the tests for curing the hepatic tumor in dogs have been carried out in a manner as will be described hereibelow.

### Tests for Curing Hepatic Tumor in Dogs:

Tweleve Beagle dogs with 4 months of age were divided into four groups of each three. The first group of the dogs was administered with physiological saline as a control group, while three groups of the dogs were administered with a carcinogenic substance. As the carcinogenic chemical substance, there was employed aflatoxin, and each dog was orally administered singly with 0.5 mg per kg, thereby causing tumor. The dogs were fed with high fat feed (containing 40% of fats). The tumor was observed with supersonic diagnosis device and confirmed by the occurrence of an abnormal high signal image (high echo image). The curing was performed by injecting the agent through a catheter into the site of the tumor, the catheter being introduced from the proper hepatic artery into high-dimensionally branched arteries at the tumor site. Before injection of the agent, a contrast medium was injected and the angiograph of the tumor was taken.

Each dog in the first group as a control was injected with 1 ml of a mixture prepared by admixing 1 ml of physiological saline with 1 ml of the contrast medium through a catheter inserted into the high-dimensionally branched arteries at the tumor site from the proper hepatic artery.

Each dog in the second group was injected with 1 ml of a mixture prepared by admixing 1 ml of the preparation formulation example No. 1 with 1 ml of the contrast medium through a catheter inserted into the high-dimensionally branched arteries at the tumor site from the proper hepatic artery.

In the third group, each dog was injected with 1 ml of a mixture prepared by admixing 1 ml of the preparation formulation example No. 2 with 1 ml of the contrast medium through a catheter inserted into the branched arteries at the tumor site from the proper hepatic artery.

In the fourth group, each dog was injected with 1 ml of a mixture prepared by admixing 1 ml of the preparation formulation example No. 6 with 1 ml of the contrast medium through a catheter inserted into the branched arteries at the tumor site from the proper hepatic artery.

Each dog in the first to fourth groups was subjected once per week (four times for four weeks in total) to abnormal high signal image diagnosis with a supersonic diagnosis device as to determine if such abnormal high signal image (high echo image) contracts or expands. On the final date of investigation in four weeks after administration, a catheter was introduced from the proper hepatic artery into the high-dimensionally branched arteries at the tumor site and the contrast medium was injected through the catheter, thereby allowing the angiography to be carried out in order to check if the angiograph has been disappeared or not. The test results are shown in Table 4 below.

**TABLE 4:**

| Tests for Curing Hepatic Tumor in Dogs | | | | |
|---|---|---|---|---|
| Group | Dog No. | Tumor Vessels | Size of Tumor | Curing Effects |
| 1 | 1 | Not changed | Expanded | None |
| | 2 | -do- | -do- | -do- |
| | 3 | -do- | -do- | -do- |
| 2 | 1 | Decreased | Contracted | Effective |
| | 2 | -do- | -do- | -do- |
| | 3 | -do- | -do- | -do- |
| 3 | 1 | -do- | -do- | -do- |
| | 2 | -do- | -do- | -do- |
| | 3 | -do- | -do- | -do- |
| 4 | 1 | Disappeared | -do- | Remarkably effective |
| | 2 | -do- | -do- | -do- |
| | 3 | -do- | -do- | -do- |

### INDUSTRIAL APPLICABILITY

The injectable solution according to the present invention can achieve local effects by selectively repairing grumous blood vessels and abnormal newborn blood vessels by allowing it to act upon vascular endotheliocytes and causing coagulation of platelets. Further, the injectable solution according to the present invention can achieve such local effects in a reversible manner by varying the concentrations of the injectable solutions. If the active ingredients of the injectable solution would be high in concentration, it can produce direct effects due to persistent vascular obstruction. On the other hand, if the active ingredients would be contained in a high concentration, the vascular obstruction becomes so reversible that the blood vessels can be repaired and that blood flow can be allowed to pass again through the vessels. As described hereinabove, the injectable solution according to the present invention can ensure the effectiveness in accordance with symptoms and sites of affected vessels by changing its concentrations. In addition, the action of the injectable solution can be slowed down, as needed, by lowering its concentrations. Furthermore, the injectable solution according to the present invention can produce the local effects by injecting it in a small amount continually into the aneurysm caused in the brain blood vessels through a catheter and by causing the composition of the injectable solution to be stayed in whirls with the blood in the swollen vascular knobs. These local effects allow the swollen portion of the aneurysm to be made matrical and then to be repaired, thereby producing normal blood vessels. As described hereinabove, the injectable solution according to the present invention is extremely effective for curing the affected abnormal tissues because it can cure the affected abnormal tissues in a non-operative way without surgery merely by injecting the curing preparations according to the present invention into the affected abnormal tissues.

The hemorrhoids of the severe grade are caused by a chronically and persistently abnormal extension of the hemorrhoidal veins, thereby causing internal hemorrhoids to occur at the upper outlet side beyond the odontoid line of the anus and external hemorrhoids to occur at the lower anal side. By injecting it directly into the branched peripheral portions of the arteriae haemorrhoidalis superior govering the hemorrhoids, the injectable solution according to the present invention can harden or fibrilate the hemorrhoids or cause the obstruction of the blood vessels, resulting in the disappearance of the hemorrhoids and eventually the recovery of the hemorrhoids.

As the tumor caused in the liver is plenty of abnormal newborn blood vessels and very active in the blood flow through the arteries, the injectable solution according to the present invention can obstruct the blood vessels of the tumor and suspend the growth of the tumor by injecting it directly into the high-dimensionally branched arteries at the tumor site through a catheter introduced thereinto from the proper hepatic artery. Once the growth of the tumor was suspended, the tumor can be allowed to disappear through the tumor cells resulting in the extremely high effects upon the curing of the affected abnormal tissues caused by the tumor.

In summary, the injectable solution according to the present invention can demonstrate the excellent curing effects upon the affected abnormal tissues, in particular by repairing the affected abnormal tissues caused by the brain aneurysm, hemorrhoids, hepatic tumor vessels and the like and causing the newborn abnormal vessels to disappear.

## Claims

1. An injectable preparation for curing affected abnormal tissues, comprising a composition containing a water-soluble aluminum compound in a concentration ranging from 0.01 mole to 0.5 mole, tannic acid at a rate of 0.5% to 25.0% with respect to said water-soluble aluminium compound, and sodium hydrogen sulfite; wherein a pH of said composition ranges from pH1.5 to pH3.5.

2. An injectable preparation as claimed in claim 1, further comprising a polyvalent alcohol or a saccharide.

3. An injectable preparation as claimed in claim 1, further comprising a chelating agent.

4. An injectable preparation as claimed in any one of claims 1 to 3, wherein said water-soluble aluminium compound is aluminium chloride, aluminium sulfate, aluminium carbonate, aluminium acetate, aluminium nitrate, aluminium lactate, aluminium tartrate, aluminium salicylate, sodium aluminium sulfate, potassium aluminium sulfate, cesium aluminium sulfate, or ammonium aluminium sulfate.

5. An injectable preparation as claimed in claim 2, wherein said polyvalent alcohol or said saccharide is mannitol, fructose, xylitol, glucose, galactose, mannose, lactose or glycerin.

6. An injectable preparation as claimed in claim 3, wherein said chelating agent is sodium citrate.

7. An injectable preparation as claimed in any one of claims 1 to 6, wherein said water-soluble aluminium compound is contained at a rate of from 0.03 mole to 0.3 mole.

8. An injectable preparation as claimed in any one of claims 1 to 7, wherein said tannic acid is contained at a rate of from 1% to 20% with respect to said water-soluble aluminium compound.

9. An injectable preparation as claimed in any one of claims 1 to 8, wherein said tannic is contained at a rate of from 0.05% to 10.0%.

10. An injectable preparation as claimed in claim 9, wherein said tannic acid is contained at a rate of from 0.1% to 5.0%.

11. An injectable preparation as claimed in any one of claims 1 to 10, wherein said sodium hydrogen sulfite is contained at a rate of from 0.05% to 0.5%.

12. An injectable preparation as claimed in claim 11, wherein said sodium hydrogen sulfite is contained at a rate of from 0.1% to 0.3%.

13. An injectable preparation as claimed in claim 2 or 5, wherein said polyvalent alcohol or saccharide is contained so as to make its osmosis from approximately three times to fifteen times the osmosis of physiological saline.

14. An injectable preparation as claimed in claim 13, wherein said polyvalent alcohol or saccharide is contained so as to make its osmosis from approximately four times to eight times the osmosis of physiological saline.

15. An injectable preparation as claimed in any one of claims 1 to 14, wherein a pH of said composition ranges from pH2 to pH3.

16. A method for the preparation of an injectable preparation according to any one of claims 1 to 15, comprising preparing a composition containing predetermined amounts of a water-soluble aluminium compound, tannic acid and sodium hydrogen sulfite as an antioxidant, making a pH of said composition from pH1.5 to pH3.5 as needed, and further preparing said composition under vacuum condition and/or in the presence of inert gases.

17. Method for the preparation of a medicament for curing affected abnormal tissues, wherein the medicament is suitable for administration through a catheter or an injection needle directly into affected abnormal tissue, the medicament comprising the injectable preparation according to any one of claims 1 to 15.

## Patentansprüche

1. Injizierbares Präparat zur Heilung von befallenen, abnormalen Geweben, das umfaßt:
Eine Zusammensetzung, die eine wasserlösliche Aluminiumverbindung in einer Konzentration im Bereich von 0,01 mol bis 0,5 mol, Gallotannin in einer Menge von 0,5 % bis 25,0 %, bezogen auf die genannte wasserlösliche Aluminiumverbindung, und Natriumhydrogensulfit enthält; wobei der pH-Wert der genannten Zusammensetzung im Bereich von 1,5 bis 3,5 liegt.

2. Injizierbares Präparat nach Anspruch 1, das weiterhin einen polyvalenten Alkohol oder ein Saccharid umfaßt.

3. Injizierbares Präparat nach Anspruch 1, das weiterhin einen Chelatbildner umfaßt.

4. Injizierbares Präparat nach irgend einem der Ansprüche 1 bis 3, worin die genannte wasserlösliche Aluminiumverbindung Aluminiumchlorid, Aluminiumsulfat, Aluminiumcarbonat, Aluminiumacetat, Aluminiumnitrat, Aluminiumlactat, Aluminiumtartrat, Aluminiumsalicylat, Natriumaluminiumsulfat, Kaliumaluminiumsulfat, Cäsiumaluminiumsulfat oder Ammoniumaluminiumsulfat ist.

5. Injizierbares Präparat nach Anspruch 2, worin der genannte polyvalente Alkohol oder das Saccharid Mannitol, Fructose, Xylitol, Glucose, Galactose, Mannose, Lactose oder Glycerin ist.

6. Injizierbares Präparat nach Anspruch 3, worin der Chelatbildner Natriumcitrat ist.

7. Injizierbares Präparat nach irgend einem der Ansprüche 1 bis 6, worin die genannte wasserlösliche Aluminiumverbindung in einer Menge von 0,03 mol bis 0,3 mol enthalten ist.

8. Injizierbares Präparat nach irgend einem der Ansprüche 1 bis 7, worin das genannte Gallotannin in einer Menge von 1 % bis 20 %, bezogen auf die genannte wasserlösliche Aluminiumverbindung, enthalten ist.

9. Injizierbares Präparat nach irgend einem der Ansprüche 1 bis 8, worin das genannte Gallotannin in einer Menge von 0,05 % bis 10 % enthalten ist.

10. Injizierbares Präparat nach Anspruch 9, worin das genannte Gallotannin in einer Menge von 0,1 bis 5 % enthalten ist.

11. Injizierbares Präparat nach irgend einem der Ansprüche 1 bis 10, worin das genannte Natriumhydrogensulfit in einer Menge von 0,05 bis 0,5 % enthalten ist.

12. Injizierbares Präparat nach Anspruch 11, worin das genannte Natriumhydrogensulfit in einer Menge von 0,1 % bis 0,3 % enthalten ist.

13. Injizierbares Präparat nach Anspruch 2 oder 5, worin der genannte polyvalente Alkohol oder das Saccharid in einer Weise enthalten ist, daß seine Osmose ungefähr dem drei- bis fünfzehnfachen der Osmose einer physiologischer Salzlösung entspricht.

14. Injizierbares Präparat nach Anspruch 13, worin der genannte polyvalente Alkohol oder das Saccharid in einer Weise enthalten ist, daß seine Osmose ungefähr dem vier- bis achtfachen der Osmose einer physiologischer Salzlösung entspricht.

15. Injizierbares Präparat nach irgend einem der Ansprüche 1 bis 14, worin der pH-Wert der genannten Zusammensetzung von pH 2 bis pH 3 beträgt.

16. Verfahren zur Herstellung eines injizierbaren Präparates nach irgend einem der Ansprüche 1 bis 15, das umfasst: die Herstellung einer Zusammensetzung, die die angegebenen Mengen einer wasserlöslichen Aluminiumverbindung, des Gallotannins und des Natriumhydrogensulfits als Antioxidationsmittel enthält, das Einstellen des pH-Werts der genannten Zusammensetzung je nach Wunsch im Bereich von pH 1,5 bis pH 3,5, und weiterhin das Herstellen der genannten Zusammensetzung unter Vakuumbedingungen und/oder in Gegenwart eines Inertgases.

17. Verfahren zur Herstellung eines Medikamentes für die Heilung befallener abnormaler Gewebe, worin das Medikament für die direkte Verabreichung in das befallene abnormale Gewebe durch einen Katheder oder eine Injektionsnadel geeignet ist, wobei das Medikament das injizierbare Präparat nach irgend einem der Ansprüche 1 bis 15 umfaßt.

## Revendications

1. Préparation injectable pour le traitement de tissus anormaux altérés, comprenant une composition contenant un composé d'aluminium soluble dans l'eau à une concentration entre 0,01 mole et 0,5 mole, de l'acide tannique à un taux de 0,5 % à 25,0 % par rapport audit composé d'aluminium soluble dans l'eau et de l'hydrogénosulfite de sodium ; le pH de ladite composition étant compris entre 1,5 et 3,5.

2. Préparation injectable selon la revendication 1, comprenant en outre un alcool polyvalent ou un saccharide.

3. Préparation injectable selon la revendication 1, comprenant en outre un agent chélatant.

4. Préparation injectable selon l'une quelconque des revendications 1 à 3, dans laquelle ledit composé d'aluminium soluble dans l'eau est le chlorure d'aluminium, le sulfate d'aluminium, le carbonate d'aluminium, l'acétate d'aluminium, le nitrate d'aluminium, le lactate d'aluminium, le tartrate d'aluminium, le salicylate d'aluminium, le sulfate de sodium et d'aluminium, le sulfate de potassium et d'aluminium, le sulfate de césium et d'aluminium ou le sulfate d'ammonium et d'aluminium.

5. Préparation injectable selon la revendication 2, dans laquelle ledit alcool polyvalent ou ledit saccharide est le mannitol, le fructose, le xylitol, le glucose, le galactose, le mannose, le lactose ou la glycérine.

6. Préparation injectable selon la revendication 3, dans laquelle ledit agent chélatant est le citrate de sodium.

7. Préparation injectable selon l'une quelconque des revendications 1 à 6, dans laquelle ledit composé d'aluminium soluble dans l'eau est contenu à un taux de 0,03 mole à 0,3 mole.

8. Préparation injectable selon l'une quelconque des revendications 1 à 7, dans laquelle ledit acide tannique est contenu à un taux de 1 % à 20 % par rapport audit composé d'aluminium soluble dans l'eau.

9. Préparation injectable selon l'une quelconque des revendications 1 à 8, dans laquelle ledit acide tannique est contenu à un taux de 0,05 % à 10,0 %.

10. Préparation injectable selon la revendication 9, dans laquelle ledit acide tannique est contenu à un taux de 0,1 % à 5,0 %.

11. Préparation injectable selon l'une quelconque des revendications 1 à 10, dans laquelle ledit hydrogénosulfite de sodium est contenu à un taux de 0,05 % à 0,5 %.

12. Préparation injectable selon la revendication 11, dans laquelle ledit hydrogénosulfite de sodium est contenu à un taux de 0,1 % à 0,3 %.

13. Préparation injectable selon la revendication 2 ou 5, dans laquelle ledit alcool polyvalent ou saccharide est contenu de telle façon qu'il donne une pression osmotique approximativement trois fois à quinze fois supérieure à la pression osmotique d'un soluté physiologique.

14. Préparation injectable selon la revendication 13, dans laquelle ledit alcool polyvalent ou saccharide est contenu de telle façon qu'il donne une pression osmotique approximativement quatre fois à huit fois supérieure à la pression osmotique d'un soluté physiologique.

15. Préparation injectable selon l'une quelconque des revendications 1 à 14, le pH de ladite composition étant compris entre 2 et 3.

16. Méthode pour la préparation d'une préparation injectable selon l'une quelconque des revendications 1 à 15, comprenant la préparation d'une composition contenant des quantités prédéterminées d'un composé d'aluminium soluble dans l'eau, d'acide tannique et d'hydrogénosulfite de sodium en tant qu'antioxydant, l'ajustement du pH de ladite composition entre 1,5 et 3,5 si nécessaire et en outre la préparation de ladite composition sous vide et/ou en présence de gaz inertes.

17. Méthode pour la préparation d'un médicament pour le traitement de tissus anormaux altérés, le médicament étant approprié à une administration par un cathéter ou par une aiguille pour injection directement dans le tissu anormal altéré, le médicament comprenant la préparation injectable selon l'une quelconque des revendications 1 à 15.
